# FASCICULE DE BREVET EUROPEEN

(11) **EP 1 492 792 B1**
(45) Date de publication et mention de la délivrance du brevet: **29.03.2006**
(21) Numéro de dépôt: 03730299.9
(22) Date de dépôt: 02.04.2003
(51) Int. Cl.: C07D 487/04, C07D 209/18, A61K 31/5025

(54) **DERIVES DE 3-HETEROARYL-3,5-DIHYDRO-4-OXO-4H-PYRIDAZINO¬4,5-B|INDOLE-1-ACETAMIDE,LEUR PREPARATION ET LEUR APPLICATION EN THERAPEUTIQUE**
3-HETEROARYL-3,5-DIHYDRO-4-OXO-4H-PYRIDAZINO¬4,5-B|INDOLACETAMIDDERIVATE, VERFAHREN ZU IHRER HERSTELLUNG UND IHRE THERAPEUTISCHE VERWENDUNG
3-HETEROARYL-3,5-DIHYDRO-4-OXO-4H-PYRIDAZINO¬4,5-B|INDOLE-1-ACETAMIDE DERIVATIVES, PREPARATION AND USE THEREOF IN MEDICAMENTS

(30) Priorité: 03.04.2002 FR 0204158
(43) Date de publication de la demande: 05.01.2005
(73) Titulaire: Sanofi-Aventis, 75013 Paris (FR)
(72) Inventeur: FROISSANT, Jacques, Cedex 981, F-41160Morée (FR); MARABOUT, Benoit, F-91300 Massy (FR); MARGUET, Frank, F-91370 Verrières Le Buisson (FR); PUECH, Frédéric, F-78170 La Celle Saint Cloud (FR)
(74) Mandataire: Ludwig, Jacques
(86) Numéro de dépôt international: PCT/FR2003/001027
(87) Numéro de publication internationale: WO 2003/082874

(56) Documents cités:
- WO-A-00/44384
- WO-A-98/15552
- WO-A-99/06406

## Description

L'invention a pour objet des composés dérivés de 3-hétéroaryl-3,5-dihydro-4-oxo-4*H*-pyridazino[4,5-*b*]indole-1-acétamide.

On connaît déjà des composés dérivés de 3,5-dihydropyridazino[4,5-*b*]indole, décrits dans les documents WO-A-9906406 et WO-A-0044384, affins *in vitro* pour les récepteurs de type périphérique aux benzodiazépines (sites p ou PBR).
Il existe toujours une nécessité de trouver et de développer des produits présentant une bonne activité *in vivo.*
L'invention répond à ce but en proposant des composés nouveaux, qui présentent une affinité *in vitro* et *in vivo* pour les récepteurs de type périphérique aux benzodiazépines.

Un premier objet de l'invention concerne les composés répondant à la formule générale (I) ci-après.
Un autre objet de l'invention concerne des procédés de préparation des composés de formule générale (I).
Un autre objet de l'invention concerne des composés utilisables notamment en tant qu'intermédiaires de synthèse des composés de formule générale (I).
Un autre objet de l'invention concerne les utilisations des composés de formule générale (1) notamment dans des médicaments ou dans des compositions pharmaceutiques.

Les composés de l'invention répondent à la formule générale (I) : 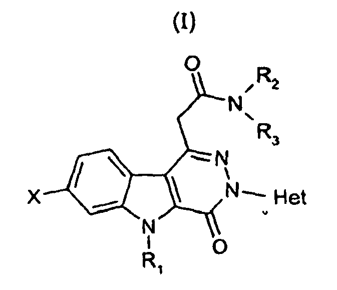 dans laquelle
X représente un atome d'hydrogène ou d'halogène,
R₁ représente un atome d'hydrogène ou un groupe (C₁-C₄)alkyle,
R₂ et R₃ représentent chacun, indépendamment l'un de l'autre, un atome d'hydrogène ou un groupe (C₁-C₄)alkyle, ou bien R₂ et R₃ forment, avec l'atome d'azote qui les porte, un groupe pyrrolidinyle, pipéridinyle, morpholinyle ou 4-(C₁-C₄)alkylpipérazinyle, et
Het représente un groupe hétéroaromatique de type pyridinyle, quinolinyle, isoquinolinyle, pyrimidinyle, pyrazinyle, pyridazinyle, le groupe hétéroaromatique pouvant porter un ou plusieurs atomes d'halogènes et/ou un ou plusieurs groupes (C₁-C₄)alkyle, (C₁-C₄)alcoxyle.

Les composés de l'invention peuvent exister à l'état de bases ou de sels d'addition à des acides. De tels sels d'addition font partie de l'invention.
Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention :
- un atome d'halogène représente un fluor, un chlore, un brome ou un iode ;
- un groupe (C₁-C₄)alkyle représente un groupe aliphatique, comportant de 1 à 4 atomes de carbone, saturé linéaire ou ramifié. A titre d'exemples, on peut citer les groupes méthyle, éthyle, propyle, isopropyle, butyle, isobutyle, terbutyle ;
- un groupe (C₁-C₄)alcoxyle représente un radical oxygène, comportant de 1 à 4 atomes de carbone, substitué par un groupe alkyle tel que défini précédemment.

Parmi les composés de formule (I) objets de l'invention, des composés préférés sont les composés pour lesquels
X représente un atome d'halogène ; et/ou
R₁ représente un (C₁-C₄)alkyle ; et/ou
R₂ et R₃ représentent chacun, indépendamment l'un de l'autre, un groupe (C₁-C₄)alkyle, ou bien R₂ et R₃ forment, avec l'atome d'azote qui les porte, un groupe pyrrolidinyle, ou 4-(C₁-C₄)alkylpipérazinyle ; et/ou
Het représente un groupe hétéroaromatique de type pyridinyle pouvant porter un ou plusieurs atomes d'halogène et/ou un ou plusieurs groupes (C₁-C₄)alkyle, (C₁-C₄)alcoxyle.

Des composés pour lesquels à la fois X, R₁, R₂, R₃ et Het sont tels que définis ci-dessus dans les sous-groupes de composés préférés, sont particulièrement préférés et plus spécifiquement parmi ceux-ci les composés pour lesquels :
X représente un atome de chlore, R₁ représente un groupe méthyle.

Parmi les composés de formule (I) objets de l'invention, à titre d'exemple, des composés de l'invention sont les suivants :
**1 :** 7-fluoro-*N*,*N*,5-triméthyl-4-oxo-3-(pyridin-2-yl)-3,5-dihydro-4*H*-pyridazino[4,5-*b*]indole-1-acetamide
**2:** chlorhydrate de 7-fluoro-*N*,*N*,5-triméthyl-4-oxo-3-(pyridin-3-yl)-3,5-dihydro-4*H-*pyridazino[4,5-*b*]indole-1-acetamide (1 :1)
**3 :** chlorhydrate de 7-fluoro-*N*,*N*,5-triméthyl-4-oxo-3-(pyridin-4-yl)-3,5-dihydro-4*H-*pyridazino[4,5-*b*]indole-1-acetamide (1 :1)
**4 :** 7-chloro-*N*,*N*,5-triméthyl-4-oxo-3-(pyridin-2-yl)-3,5-dihydro-4*H*-pyridazino[4,5-*b*]indole-1-acetamide
**5** : 7-chloro-*N*,*N*,5-triméthyl-4-oxo-3-(pyridin-3-yl)-3,5-dihydro-4*H*-pyridazino[4,5-*b*]indole-1-acetamide
**6** : chlorhydrate de 7-chloro-*N*,*N*,5-triméthyl-4-oxo-3-(pyridin-3-yl)-3,5-dihydro-4*H-*pyridazino[4,5-*b*]indole-1-acetamide (1 :1)
**7** : chlorhydrate de 7-chloro-*N*,*N*,5-triméthyl-4-oxo-3-(pyridin-4-yl)-3,5-dihydro-4*H-*pyridazino[4,5-*b*]indole-1-acetamide (1 :1)
**8** : 7-chloro-*N*,*N*,5-triméthyl-4-oxo-3-(6-méthylpyridin-3-yl)-3,5-dihydro-4*H*-pyridazino[4,5-*b*]indole-1-acetamide
**9** : 7-chloro-*N,N*-diéthyl-5-méthyl-4-oxo-3-(pyridin-3-yl)-3,5-dihydro-4*H*-pyridazino[4,5-*b*]indole-1-acetamide
**10**: chlorhydrate de 4-méthyl-1-[2-[7-chloro-5-méthyl-3-(pyridin-3-yl)-4-oxo-3,5-dihydro-4*H*-pyridazino[4,5-*b*]indol-1-yl]acet-1-yl]pipérazine (1:1)
**11**: 1-[2-[7-chloro-5-méthyl-3-(pyridin-3-yl)-4-oxo-3,5-dihydro-4*H*-pyridazino[4,5-*b*]indol-1-yl]acet-1-yl]pyrrolidine
**12**: chlorhydrate de 1-[2-[7-chloro-5-méthyl-3-(pyridin-4-yl)-4-oxo-3,5-dihydro-4*H-*pyridazino[4,5-*b*]indol-1-yl]acet-1-yl]pyrrolidine (1:1)

Les composés de formule générale (I) peuvent être préparés par des procédés illustrés dans ce qui suit.

Dans toute la suite de la description, les composés intermédiaires (II), (III), (IV) et (V) sont ceux présentés dans le schéma ci-après.

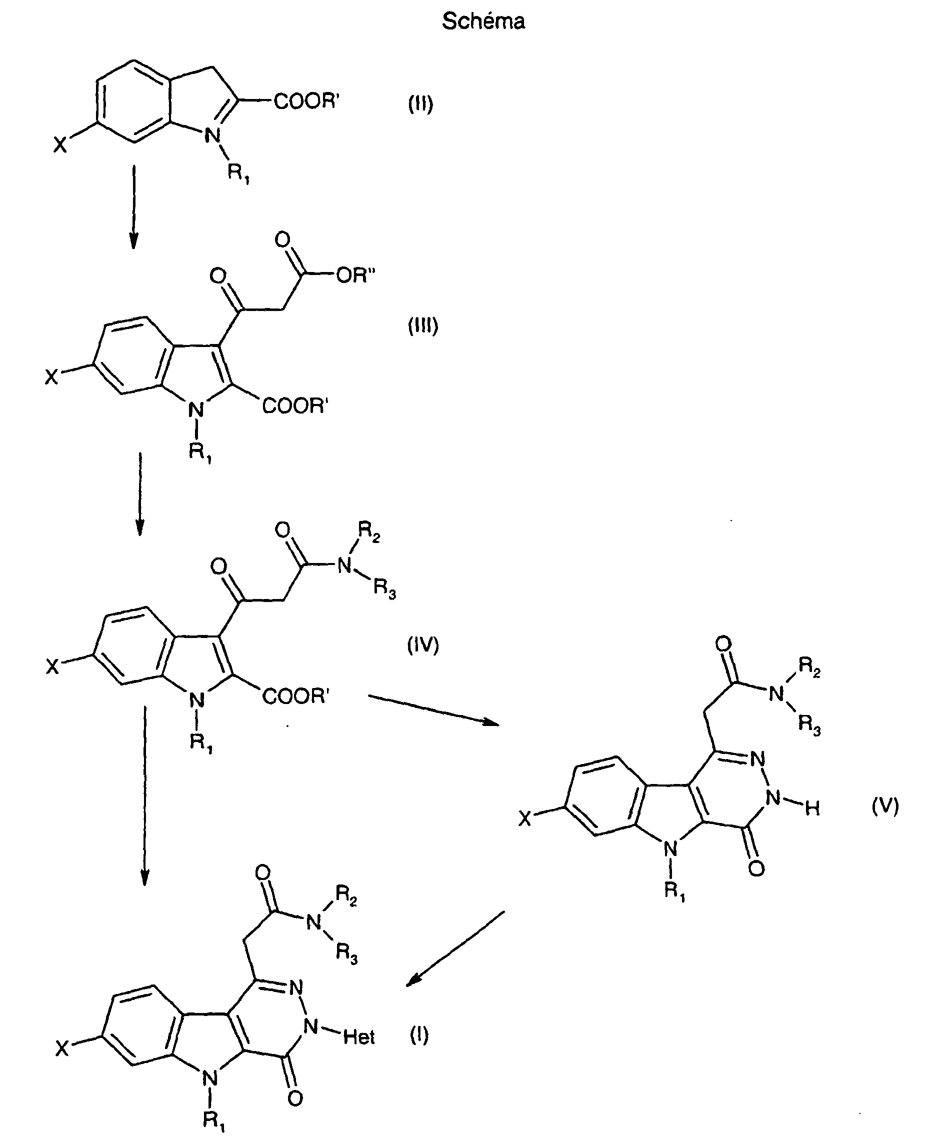

Un composé de formule générale (II), dans laquelle X et R₁ sont tels que définis ci-dessus et R' représente un groupe (C₁-C₄)alkyle, est traité avec un 3-chloro-3-oxopropanoate de formule générale ClCOCH₂CO₂R", dans laquelle R" représente un groupe (C₁-C₄)alkyle, dans un solvant tel que le dichloroéthane, à température ambiante, en présence d'un acide de Lewis, par exemple le tétrachlorure de titane, pour obtenir le diester de formule générale (III).
La fonction cétoester du diester de formule générale (III) est transformée en cétoamide pour donner le composé de formule générale (IV) par action d'une amine de formule générale HNR₂R₃, dans laquelle R₂ et R₃ sont tels que définis ci-dessus, en présence d'un catalyseur comme la 4-(diméthylamino)pyridine.
Selon un première voie de préparation, le composé de formule générale (IV) est traité dans un solvant polaire en présence d'acide, avec une hétéroarylhydrazine, pour obtenir un amide de formule générale (I).
Selon une deuxième voie de préparation, le composé de formule générale (IV) est traité avec de l'hydrazine en chauffant dans un solvant comme le toluène en présence d'une quantité catalytique d'acide pour obtenir un pyridazinoindole de formule générale (V). Finalement, une réaction de *N*-arylation est effectuée sur le pyridazinoindole de formule générale (V) en présence d'un halogénure d'hétéroaryle, ou bien d'un dérivé d'acide hétéroarylboronique et d'un sel métallique tel que un sel de cuivre conduisant à un composé de formule générale (I).
Les réactifs employés ci-dessus sont disponibles dans le commerce ou décrits dans la littérature, ou bien peuvent être préparés selon des méthodes qui y sont décrites ou qui sont connues de l'Homme du métier.
Plus particulièrement, les dérivés d'acides boroniques portant un groupe hétéroaromatique peuvent être préparés par des méthodes analogues à celles connues dans la littérature *(Synth. Commun.* **1996,** *26*, 3543 et WO9803484).

La préparation des composés de départ de formule générale (II) est décrite dans le document WO-A-0044751 dans le cas où X est un atome de chlore. Dans le cas où X est un atome de fluor, le composé de formule générale (II) est préparé de façon analogue à partir du 6-fluoroindole-2-carboxylate de méthyle, décrit dans la littérature (*J*. *Med. Chem.* **2000,** *43,* 4701).

Un autre objet de l'invention concerne les composés de formule générale (III) 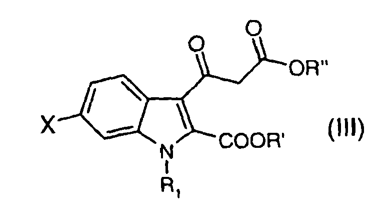 dans laquelle
X représente un atome d'hydrogène ou d'halogène,
R₁ représente un atome d'hydrogène ou un groupe (C₁-C₄)alkyle,
R' et R" représentent chacun, indépendamment l'un de l'autre, un groupe (C₁-C₄)alkyle, utiles en tant qu'intermédiaires de synthèse pour la préparation des composés de formule générale (I).

Un autre objet de l'invention concerne les composés de formule générale (IV) 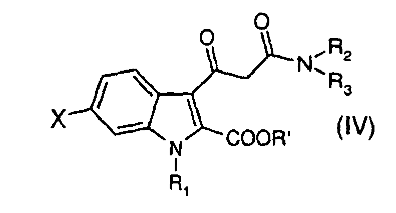 dans laquelle
X représente un atome d'hydrogène ou d'halogène,
R₁ représente un atome d'hydrogène ou un groupe (C₁-C₄)alkyle,
R' représente un groupe (C₁-C₄)alkyle,
R₂ et R₃ représentent chacun, indépendamment l'un de l'autre, un atome d'hydrogène ou un groupe (C₁-C₄)alkyle, ou bien R₂ et R₃ forment, avec l'atome d'azote qui les porte, un groupe pyrrolidinyle, pipéridinyle, morpholinyle ou 4-(C₁-C₄)alkylpipérazinyle,
utiles en tant qu'intermédiaires de synthèse pour la préparation des composés de formule générale (I).

Un autre objet de l'invention concerne les composés de formule générale (V) 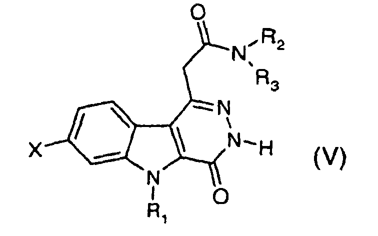 dans laquelle
X représente un atome d'hydrogène ou d'halogène,
R₁ représente un atome d'hydrogène ou un groupe (C₁-C₄)alkyle,
R₂ et R₃ représentent chacun, indépendamment l'un de l'autre, un atome d'hydrogène ou un groupe (C₁-C₄)alkyle, ou bien R₂ et R₃ forment, avec l'atome d'azote qui les porte, un groupe pyrrolidinyle, pipéridinyle, morpholinyle ou 4-(C₁-C₄)alkylpipérazinyle,
utiles en tant qu'intermédiaires de synthèse pour la préparation des composés de formule générale (I).

Les exemples qui vont suivre illustrent la préparation de quelques composés de l'invention. Ces exemples ne sont pas limitatifs et ne font qu'illustrer l'invention. Les numéros des composés exemplifiés renvoient à ceux donnés dans le tableau ci-après, qui illustre les structures chimiques et les propriétés physiques de quelques composés suivant l'invention. Les microanalyses élémentaires, et les spectres I.R. et R.M.N. confirment les structures des composés obtenus.

### Exemple 1 (Composé N°1).

### 7-fluoro-N,N,5-triméthyl-4-oxo-3-(pyridin-2-yl)-3,5-dihydro-4H-pyridazino[4,5-b]indole-1-acétamide

### 1.1. 6-fluoro-1-méthyl-1H-indole-2-carboxylate de méthyle

On agite pendant 2 h à température ambiante une suspension de 7,9 g (197 mmol) d'hydrure de sodium à 60% (préalablement lavé à l'éther de pétrole) et de 36,1 g (176 mmol) de 6-fluoro-1*H*-indole-2-carboxylate de méthyle (contenant 10 à 20% de 6-fluoro-1*H*-indole-2-carboxylate d'éthyle) dans 250 ml de *N,N*-diméthylformamide. On ajoute ensuite 12 ml (193 mmol) de iodométhane dans 50 ml de *N,N*-diméthylformamide et on agite le mélange à température ambiante pendant 12 h.
On verse le contenu dans un mélange eau et glace. On additionne du dichlorométhane et on neutralise la phase aqueuse avec de l'acide chlorhydrique (1 N). On décante la phase organique, on la lave à l'eau, on la sèche sur sulfate de sodium, on filtre et on concentre sous pression réduite. On purifie le résidu par chromatographie sur colonne de gel de silice dans un mélange de solvants (cyclohexane/dichlorométhane : 50/50 à 0/100 puis dichlorométhane/acétate d'éthyle : 100/0 à 70/30).
On isole 32,7 g (170 mmol) d'un composé blanc de 6-fluoro-1-méthyl-1*H*-indole-2-carboxylate de méthyle contenant 10 à 20% de 6-fluoro-1-méthyl-1*H*-indole-2-carboxylate d'éthyle.

### 1.2. 3-[6-fluoro-2-(méthoxycarbonyl)-1-méthyl-1H-indol-3-yl]-3-oxopropanoate de méthyle

On ajoute par portions 6,5 ml (60 mmol) de 3-chloro-3-oxopropanoate de méthyle à une solution de 6,6 ml (60 mmol) de tétrachlorure de titane dans 80 ml de 1,2-dichloroéthane. On agite pendant 30 min à température ambiante. On ajoute une solution de 5 g (24,1 mmol) de 6-fluoro-1-méthyl-1*H*-indole-2-carboxylate de méthyle (contenant 10 à 20% de 6-fluoro-1-méthyl-1*H*-indole-2-carboxylate d'éthyle), obtenu à l'étape 1.1., et on agite pendant 20 h à 40°C. On verse le mélange dans de l'eau glacée et on extrait au dichlorométhane. On décante la phase organique, on la lave à l'eau, on la sèche sur sulfate de sodium, on filtre et on concentre sous pression réduite. On purifie le résidu par chromatographie sur colonne de gel de silice (éluant : cyclohexane/dichlorométhane: 90/10 à 0/100 puis dichlorométhane/acétate d'éthyle: 100/0 à 50/50).
On obtient 13 g d'un solide pâteux contenant majoritairement le composé. Il est engagé tel que dans la suite de la synthèse.

### 1.3. N,N-diméthyl-3-[6-fluoro-2-(méthoxycarbonyl)-1-méthyl-1H-indol-3-yl]-3-oxopropanamide

On fait passer un courant de diméthylamine gazeuze dans un mélange de 13 g (44,4 mmol) de 3-[6-fluoro-2-(méthoxycarbonyl)-1-méthyl-1*H*-indol-3-yl]-3-oxopropanoate de méthyle, obtenu à l'étape 1.2., et de 0,2 g (1,63 mmol) de 4-(*N,N*-diméthyl)aminopyridine dans 80 ml de toluène. On adapte aussitôt un réfrigérant surmonté d'un ballon de baudruche et on agite la solution à 100°C durant 20 h. On refroidit le mélange à température ambiante, on concentre sous pression réduite. On ajoute 200 ml de dichlorométhane, de l'eau et de l'acide chlorhydrique (1N). On décante la phase organique, on la lave à l'eau, on la sèche sur sulfate de sodium, on filtre et on concentre sous pression réduite. On purifie le résidu par chromatographie sur colonne de gel de silice (éluant : cyclohexane/dichlorométhane : 50/50, puis dichlorométhane/acétate d'éthyle: 100/0 à 0/100).
On isole 4,6 g (14 mmol) d'un solide jaune engagé tel quel dans la suite de la synthèse.

### 1.4. 7-fluoro-N,N,5-triméthyl-4-oxo-3-(pyrid-2-yl)-3,5-dihydro-4H-pyridazino[4,5-b]indole-1-acétamide

On porte au reflux pendant 22 h une solution de 1,4 g (4,1 mmol) de *N*,*N*-diméthyl-3-[6-fluoro-2-(méthoxycarbonyl)-1-méthyl-1*H*-indol-3-yl]-3-oxopropanamide, obtenu à l'étape 1.3., dans 40 ml d'éthanol absolu, avec quelques gouttes d'acide acétique glacial et 1,4 g (12,8 mmol) de 2-pyridylhydrazine.
On refroidit le milieu et on concentre sous pression réduite. On ajoute de l'eau et 200 ml de dichlorométhane. On ajoute une solution de soude jusqu'à pH > 10. On décante la phase organique, on la lave à l'eau, on la sèche sur sulfate de sodium, on filtre et on concentre sous pression réduite. On purifie le résidu par chromatographie sur colonne de gel de silice dans un mélange de solvants (dichlorométhane/acétate d'éthyle : 100/0 à 0/100. puis acétate d'éthyle/méthanol : 100/0 à 90/10). Le produit obtenu est ensuite chromatographié sur colonne d'alumine neutre dans un mélange de solvants (dichlorométhane/acétate d'éthyle : 100/0 à 0/100, puis acétate d'éthyle/méthanol : 100/0 à 90/10). On obtient un solide qui est rincé avec de l'éther diéthylique.
On isole 0,25 g (0,66 mmol) de composé sous forme d'un solide blanc.
Point de fusion : 222 - 223°C ; M+H⁺ : 380.

### Exemple 2 (Composé N°6).

### chlorhydrate de 7-chloro-N,N,5-triméthyl-4-oxo-3-(pyridin-3-yl)-3,5-dihydro-4H-pyridazino[4,5-b]indole-1-acétamide

### 2.1. 3-[6-chloro-2-(méthoxycarbonyl)-1-méthyl-1H-indol-3-yl]-3-oxopropanoate d'éthyle

On refroidit à 0°C une solution de 6,2 ml (48,4 mmol) de 3-chloro-3-oxopropanoate d'éthyle dans 70 ml de 1,2-dichloroéthane. On ajoute par petites portions 5,3 ml (48,3 mmol) de tétrachlorure de titane et on agite pendant 30 min à 0°C. On ajoute une solution de 4,3 g (19,2 mmol) de 6-chloro-1-méthyl-*1H*-indole-2-carboxylate de méthyle dans 35 ml de 1,2-dichloroéthane et on agite pendant 12 h à température ambiante. On verse le mélange dans de l'eau glacée et on extrait au dichlorométhane. On décante la phase organique, on la lave à l'eau, on la sèche sur sulfate de sodium, on filtre et on concentre sous pression réduite. On purifie le résidu par chromatographie sur colonne de gel de silice (éluant : cyclohexane/acétate d'éthyle: 90/10 à 80/20). On obtient un solide jaune que l'on triture dans de l'heptane puis dans de l'éther diisopropylique.
On récupère 2,84 g (8,4 mmol) de composé sous forme d'un solide couleur crème.

### 2.2. 3-[6-chloro-2-(méthoxycarbonyl)-1-méthyl- 1H-indol-3-yl]-N,N-diméthyl-3-oxopropanamide

On fait passer un courant de diméthylamine gazeuze dans un mélange de 15 g (44,4 mmol) de 3-[6-chloro-2-(méthoxycarbonyl)-1-méthyl-1*H*-indol-3-yl]-3-oxopropanoate d'éthyle, obtenu à l'étape 2.1., et de 0,2 g (1,63 mmol) de 4-(*N,N*-diméthyl)aminopyridine dans 100 ml de toluène. On adapte aussitôt un réfrigérant surmonté d'un ballon de baudruche et on agite la solution à 100°C sous faible pression durant 20 h. On refroidit le mélange à température ambiante, on concentre sous pression réduite et on chromatographie le résidu sur colonne de gel de silice (éluant : cyclohexane/dichlorométhane : 50/50, puis dichlorométhane/acétate d'éthyle: 100/0 à 0/100). On obtient 3,8 g d'un solide jaune que l'on recristallise dans un mélange dichlorométhane/acétate d'éthyle.
On isole 1,8 g (5,3 mmol) d'un solide blanc-jaune.

### 2.3. 7-chloro-N,N,5-triméthyl-4-oxo-3,5-dihydro-4H-pyridazino[4,5-b]indole-1-acétamide On porte à 90°C pendant 24 h une solution de 1,7 g (5,2 mmol) de 3-[6-chloro-2-(méthoxycarbonyl)-1-méthyl-1H-indol-3-yl]-N,N-diméthyl-3-oxopropanamide, obtenu à l'étape 2.2., dans 150 ml de toluène, en présence de 1,8 ml (36,8 mmol) d'hydrazine monohydrate et d'une quantité catalytique d'acide p-toluènesulfonique.

On refroidit le milieu, on recueille un insoluble par filtration, on le lave avec de l'eau, puis de l'éther diisopropylique et on le sèche sous pression réduite.
On isole 1,70 g (5,2 mmol) de composé sous forme d'un solide blanc.
Point de fusion : > 300°C.

### 2.4. chlorhydrate de 7-chloro-N,N,5-triméthyl-4-oxo-3-(pyridin-3-yl)-3,5-dihydro-4H-pyridazino[4,5-b]indole-1-acétamide

On solubilise 0,2 g (0,63 mmol) de 7-chloro-*N*,*N*,5-triméthyl-4-oxo-3,5-dihydro-4*H-*pyridazino[4,5-*b*]indole-1-acétamide, obtenu à l'étape 2.3., dans 15 ml de *N-*méthylpyrrolidone. A température ambiante et sous atmosphère d'argon, on introduit 0,11 ml (1,4 mmol) de pyridine, 0,19 ml (1,4 mmol) de triéthylamine, 1 g de tamis moléculaire, 0,24 g (1,3 mmol) d'acétate cuivrique et 0,22 g (1,4 mmol) de 2-(pyridin-3-yl)-1,3,2-dioxaborinane. Après 24 h de réaction, on élimine les insolubles par filtration et on ajoute à la solution 0,11 ml (1,4 mmol) de pyridine, 0,19 ml (1,4 mmol) de triéthylamine, 1 g de tamis moléculaire, 0,24 g (1,3 mmol) d'acétate cuivrique et 0,22 g (1,4 mmol) de 2-(pyridin-3-yl)-1,3,2-dioxaborinane. On agite la réaction pendant 24 h supplémentaires. On élimine les insolubles par filtration et on concentre sous pression réduite pour éliminer le solvant. On ajoute du dichlorométhane et de l'eau. On extrait la phase aqueuse au dichlorométhane. On réunit les phases organiques et on les lave à l'eau. On sèche sur sulfate de sodium, on filtre et on concentre sous pression réduite. On purifie le résidu par chromatographie sur colonne de gel de silice (éluant : dichlorométhane, puis acétate d'éthyle/méthanol: 100/0 à 80/20). On obtient un solide que l'on dissout dans un mélange dichlorométhane/méthanol. On ajoute de l'acétate d'éthyle et on concentre partiellement le mélange. On isole un solide par filtration que l'on recristallise dans un mélange d'éthanol et de dichlorométhane. On récupère 110 mg de 7-chloro-*N,N*,5-triméthyl-4-oxo-3-(pyridin-3-yl)-3,5-dihydro-4*H*-pyridazino[4,5-*b*]indole-1-acétamide (composé n°5) sous forme d'un solide blanc.
Point de fusion : 255 - 256°C.
On forme le chlorhydrate par dissolution du solide isolé ci-dessus dans un mélange de méthanol et de dichlorométhane et par ajout d'une solution 5N d'acide chlorhydrique dans du propan-2-ol. Après recristallisation dans de l'éthanol, on isole 0,09 g (0,20 mmol) de composé sous forme de solide blanc.
Point de fusion : 250 - 252°C; M+H⁺ : 396.

### Exemple 3 (Composé N°10).

### Chlorhydrate de 4-méthyl-1-[2-[7-chloro-5-méthyl-4-oxo-3-(pyridin-3-yl)-3,5-dihydro-4H-pyridazino[4,5-b]indol-1-yl]acet-1-yl]pipérazine (1:1)

### 3.1. [3-[6-chloro-2-(méthoxycarbonyl)-1-méthyl-1H-indol-3-yl]3-oxopropion-1-yl]4-méthylpipérazine

On porte au reflux pendant 12 h une solution de 2,84 g (8,4 mmol) de 3-[6-chloro-2-(méthoxycarbonyl)-1-méthyl-*1H*-indol-3-yl]-3-oxopropanoate d'éthyle, obtenu à l'étape 2.1. de l'exemple 2, dans 160 ml de toluène, en présence de 3,7 ml (34 mmol) de *N-*méthylpipérazine et de 110 mg (0,9 mmol) de 4-(*N*,*N*-diméthylamino)pyridine.

On refroidit le mélange à température ambiante. On ajoute 100 ml de dichlorométhane, 80 ml d'eau et 10 ml d'une solution aqueuse d'ammoniaque à 20%. On décante la phase organique, on extrait la phase aqueuse avec du dichlorométhane (2 fois 100 ml), on réunit les phases organiques. On lave à l'eau, on sèche sur sulfate de sodium, on filtre, on concentre sous pression réduite et on purifie le résidu par chromatographie sur colonne de gel de silice (éluant : dichlorométhane/méthanol : 100/0 à 90/10). On obtient 1,68 g (4,3 mmol) d'une huile jaune.

### 3.2. 4-Méthyl-1-[2-[7-chloro-5-méthyl-4-oxo-3, 5-dihydro-4H-pyridazino[4,5-b]indol-1-yl]acet-1-yl]pipérazine

On porte à 90°C pendant 24 h une solution de 1,68 g (4,3 mmol) de [3-[6-chloro-2-(méthoxycarbonyl)-1-méthyl-1*H*-indol-3-yl]3-oxopropion-1-yl]4-méthylpipérazine, obtenu à l'étape 3.1., dans 80 ml de toluène, en présence de 1,7 ml (35 mmol) d'hydrazine monohydrate et d'une quantité catalytique d'acide *p*-toluènesulfonique.
On refroidit le milieu, on recueille un insoluble par filtration, on le lave avec de l'eau, puis de l'éther diisopropylique et on le sèche sous pression réduite.
On isole 1,43 g (3,8 mmol) de composé sous forme d'un solide blanc.
Point de fusion : > 300°C.

### 3.3. Chlorhydrate de 4-méthyl-1-[2-[7-chloro-5-méthyl-4-oxo-3-(pyridin-3-yl)-3,5-dihydro-4H-pyridazino[4,5-b]indol-1-yl]acet-1-yl]pipérazine (1:1)

On solubilise 0,45 g (1,2 mmol) de 4-méthyl-1-[2-[7-chloro-5-méthyl-4-oxo-3,5-dihydro-4*H*-pyridazino[4,5-*b*]indol-1-yl]acet-1-yl]pipérazine, obtenu à l'étape 3.2., dans 30 ml de *N*-méthylpyrrolidone. A température ambiante et sous atmosphère d'argon, on introduit 0,2 ml (2,4 mmol) de pyridine, 0,34 ml (2,4 mmol) de triéthylamine, 0,30 g de tamis moléculaire, 0,44 g (2,4 mmol) d'acétate cuivrique et 0,39 g (2,4 mmol) de 2-(pyridin-3-yl)-1,3,2-dioxaborinane. Après 24 h de réaction, on élimine les insolubles par filtration et on ajoute à la solution 0,2 ml (2,4 mmol) de pyridine, 0,34 ml (2,4 mmol) de triéthylamine, 0,30 g de tamis moléculaire, 0,44 g (2,4 mmol) d'acétate cuivrique et 0,39 g (2,4 mmol) de 2-(pyridin-3-yl)-1,3,2-dioxaborinane. On agite la réaction pendant 24 h supplémentaires. On élimine les insolubles par filtration et on concentre sous pression réduite pour éliminer le solvant. On ajoute du dichlorométhane et de l'eau. On extrait la phase aqueuse au dichlorométhane. On réunit les phases organiques et on les lave à l'eau. On sèche sur sulfate de sodium et on concentre sous pression réduite. On purifie le résidu par chromatographie sur colonne de silice (éluant : dichlorométhane/méthanol: 100/0 à 90/10).
On récupère un solide blanc dont on forme le chlorhydrate par solubilisation dans un mélange de propan-2-ol et de méthanol et par ajout d'une solution 0,1 N d'acide chlorhydrique dans le propan-2-ol. Après recristallisation dans un mélange de propan-2-ol et de méthanol, on isole 0,34 g (0,70 mmol) de composé sous forme de solide blanc.
Point de fusion : 287°C (décomposition); M+H⁺ : 451.

### Exemple 4 (Composé N°8).

### 7-chloro-N,N,5-triméthyl-4-oxo-3-(6-méthylpyridin-3-yl)-3,5-dihydro-4H-pyridazino[4,5-b]indole-1-acétamide

On solubilise 0,4 g (1,25 mmol) de 7-chloro-*N,N*,5-triméthyl-4-oxo-3,5-dihydro-4*H-*pyridazino[4,5-*b*]indole-1-acétamide, obtenu à l'étape 2.3. de l'exemple 2, dans 45 ml de *N*-méthylpyrrolidone. A température ambiante et sous atmosphère d'argon, on introduit 0,2 ml (2,5 mmol) de pyridine, 0,35 ml (2,5 mmol) de triéthylamine, 0,40 g de tamis moléculaire, 0,45 g (2,5 mmol) d'acétate cuivrique et 0,80 g (3,6 mmol) de 4,4,5,5-tétraméthyl-2-(6-méthylpyridin-3-yl)-1,3,2-dioxaborolane. Après 24 h de réaction, on ajoute à la solution 0,2 ml (2,5 mmol) de pyridine, 0,35 ml (2,5 mmol) de triéthylamine, 0,40 g de tamis moléculaire, 0,45 g (2,5 mmol) d'acétate cuivrique et 0,80 g (3,6 mmol) de 4,4,5,5-tétraméthyl-2-(6-méthylpyridin-3-yl)-1,3,2-dioxaborolane. On agite la réaction pendant 24 h supplémentaires. On élimine les insolubles par filtration et on concentre le filtrat sous pression réduite pour éliminer le solvant. On ajoute du dichlorométhane et de l'eau. On extrait la phase aqueuse au dichlorométhane. On réunit les phases organiques et on les lave à l'eau. On sèche sur sulfate de sodium, on filtre et on concentre sous pression réduite. On purifie le résidu par chromatographie sur colonne de gel de silice (éluant : dichlorométhane/méthanol: 100/0 à 80/20). On obtient un solide que l'on recristallise dans un mélange isopropanol/méthanol.
On isole 0,12 g (0,29 mmol) de composé sous forme de solide blanc.
Point de fusion : 253 - 255°C; M+H⁺ : 410.

Le tableau qui suit illustre les structures chimiques et les propriétés physiques de quelques composés de l'invention.
Dans la colonne "Sel" de ces tableaux, "HCl" désigne un chlorhydrate, "-" désigne un composé à l'état de base. Les rapports molaires acide : base sont indiqués en regard.
L'abréviation dec. signifie qu'à la température donnée, le solide est à l'état de décomposition.

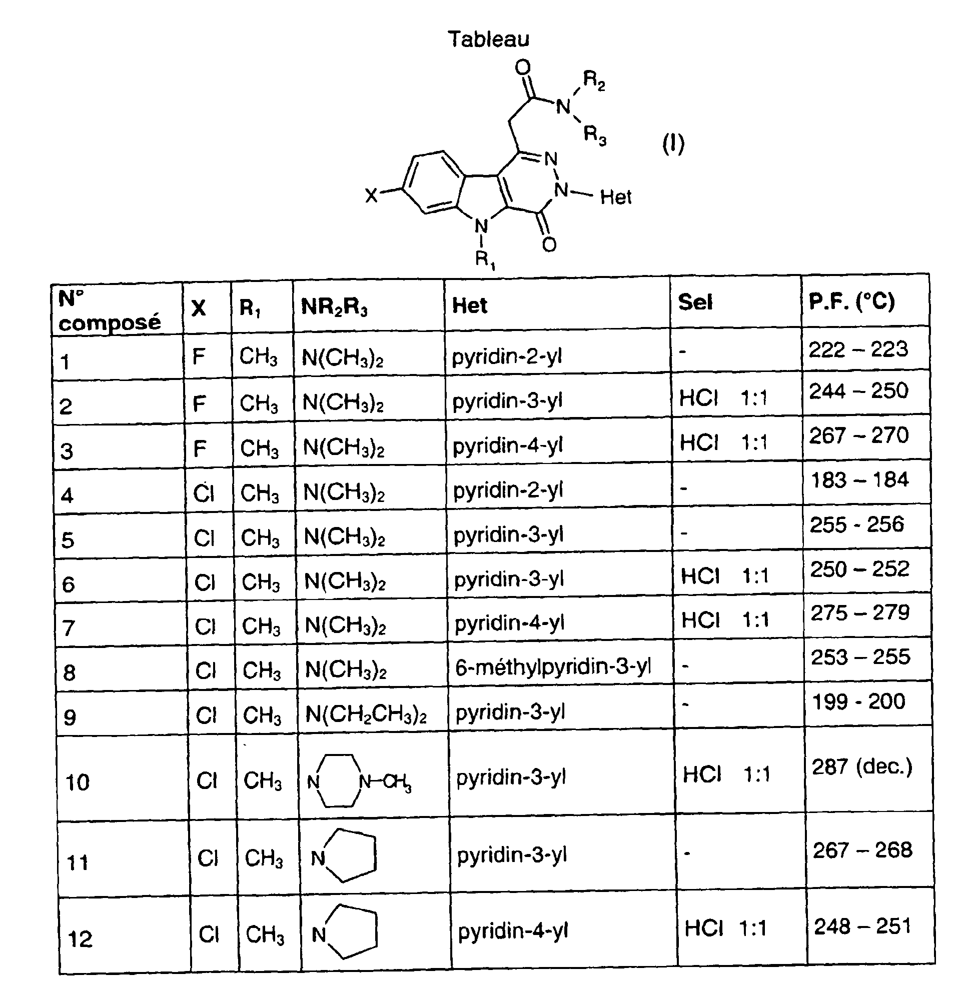

Les composés de l'invention ont fait l'objet d'essais pharmacologiques qui ont mis en évidence leur intérêt comme substances à activités thérapeutiques.

Les composés de l'invention présentent également des caractéristiques de solubilité dans l'eau qui favorisent une bonne activité *in vivo.*

### Etude de la liaison [³H]Ro5-4864 aux récepteurs de type périphérique aux benzodiazépines (sites p ou PBR).

L'affinité des composés de l'invention pour les sites p ou PBR (sites de liaison de type périphérique aux benzodiazépines) a été déterminée.

Les récepteurs sites p peuvent être marqués sélectivement dans des membranes de rein de rat incubées en présence de [³H]Ro5-4864. Les composés de l'invention ont fait l'objet d'une étude *in vitro* quant à leur affinité pour ces récepteurs.
Les animaux utilisés sont des rats mâles Sprague Dawley (Iffa Credo) de 180 à 300 mg. Après décapitation, le rein est prélevé et le tissu est homogénéisé à 4°C au moyen d'un homogénéiseur Polytron™ pendant 2 min à 6/10 de la vitesse maximale, dans 35 volumes de tampon phosphate Na₂HPO₄ 50 mM, à un pH ajusté à 7,5 avec du NaH₂PO₄. L'homogénat membranaire est filtré sur gaze et dilué 10 fois avec du tampon.
Le [³H]Ro5-4864 (Activité spécifique : 70-90 Ci/mmol ; New England Nuclear), à une concentration de 0,5 nM, est incubé en présence de 100 µl de l'homogénat membranaire dans un volume final de 1 ml de tampon contenant le composé à tester.
Après une incubation de 3 h à 0°C, les membranes sont récupérées par filtration sur filtres Whatman GF/B™ lavés avec 2 fois 4,5 ml de tampon d'incubation froid (0°C). La quantité de radioactivité retenue par le filtre est mesurée par scintigraphie liquide.
Pour chaque concentration de composé étudié, le pourcentage d'inhibition de la liaison du [³H]Ro5-4864, puis la concentration Cl₅₀, concentration qui inhibe 50% de la liaison spécifique, sont déterminés.
Les Cl₅₀ des meilleurs composés de l'invention présentent des valeurs allant de 1 nM à 200 nM.
Les composés de l'invention sont donc des ligands affins pour les récepteurs de type périphérique aux benzodiazépines.

### Etude de l'activité neurotrophe.

### Test de survie des motoneurones après section du nerf facial chez le rat âgé de 4 jours.

Après lésion du nerf facial chez le rat immature, les motoneurones du noyau facial subissent une mort neuronale par apoptose. L'évaluation de la survie neuronale est réalisée à l'aide de méthodes histologiques et comptage neuronal.
Des rats immatures de 4 jours sont anesthésiés au pentobarbital (3 mg/kg par voie i.p.).
Le nerf facial droit est dégagé et sectionné, à sa sortie du foramen stylomastoïdien.
Après le réveil, les ratons sont remis avec leur mère et traités, pendant 7 jours, par une ou deux administrations quotidiennes, par voie orale ou intrapéritonéale, à des doses allant de 1 à 10 mg/kg.
7 jours après la lésion, les animaux sont décapités, et les cerveaux congelés dans l'isopentane à -40°C. Le noyau facial est coupé au cryostat, en sections de 10 µm, dans sa totalité. Les motoneurones sont colorés au crésyl violet et comptés à l'aide du logiciel Histo™ (Biocom™).
Dans ce modèle, les composés de l'invention augmentent la survie neuronale d'environ 10 à 30%.

Les résultats des essais montrent que les composés de l'invention favorisent la régénération nerveuse.

Les composés selon l'invention peuvent donc être utilisés pour la préparation de médicaments.

Ainsi, selon un autre de ses aspects, l'invention a pour objet des médicaments qui comprennent un composé de formule (I), ou un sel d'addition de ce dernier à un acide pharmaceutiquement acceptable, ou encore un hydrate ou un solvat du composé de formule (I).

Ces médicaments trouvent leur emploi en thérapeutique, notamment pour la prévention et/ou le traitement des neuropathies périphériques de différent types, comme les neuropathies traumatiques ou ischémiques, neuropathies infectieuses, alcooliques, médicamenteuses ou génétiques, ainsi que des affections du motoneurone, telle que les amyotrophies spinales et la sclérose latérale amyotrophique. Ces médicaments trouveront également une application dans le traitement des maladies neurodégénératives du système nerveux central, soit de type aigu comme les accidents vasculaires cérébraux et les traumatismes crâniens et médullaires, soit de type chronique comme les maladies auto-immunes (sclérose en plaques), la maladie d'Alzheimer, la maladie de Parkinson et toute autre maladie dans laquelle l'administration de facteurs neurotrophes est censée avoir un effet thérapeutique.

Les composés selon l'invention peuvent aussi être utilisés dans les traitements de l'insuffisance rénale aiguë ou chronique, de la glomérulonéphrite, de la néphropathie diabétique, de l'ischémie et de l'insuffisance cardiaques, de l'infarctus du myocarde, de l'ischémie des membres inférieurs, du vasospasme coronaire, de l'angine de poitrine, des pathologies associées aux valves cardiaques, des maladies cardiaques inflammatoires, des effets secondaires dus à des médicaments cardiotoxiques ou aux suites d'une chirurgie cardiaque, de l'athérosclérose et de ses complications thromboemboliques, de la resténose, des rejets de greffes, des conditions liées à une prolifération ou une migration incorrectes des cellules musculaires lisses.

Par ailleurs, des données récentes de la littérature indiquent que le récepteur de type périphérique aux benzodiazépines pourrait jouer un rôle fondamental dans la régulation de la prolifération cellulaire et les processus de cancérisation. D'une manière générale, et par comparaison avec des tissus normaux, une densité accrue de récepteurs de type périphérique aux benzodiazépines est observée dans différents types de tumeurs et cancers.

Dans les astocytomes humains, le niveau d'expression du récepteur de type périphérique aux benzodiazépines est corrélé avec le degré de malignité de la tumeur, l'index de prolifération et la survie des patients. Dans les tumeurs cérébrales humaines, l'augmentation du nombre de récepteurs de type périphérique aux benzodiazépines est utilisée comme une indication diagnostique en imagerie médicale et comme cible thérapeutique pour des conjugués formés d'un ligand du récepteur de type périphériques aux benzodiazépines et d'une drogue cytostatique. Une densité élevée de récepteurs de type périphérique aux benzodiazépines est également observée dans les carcinomes ovariens et les cancers du sein. Concernant ces derniers, il a été démontré que le niveau d'expression des récepteurs de type périphérique aux benzodiazépines est relié au potentiel agressif de la tumeur ; de plus la présence d'un agoniste du récepteur de type périphérique aux benzodiazépines stimule la croissance d'une lignée de cancer mammaire.

L'ensemble de ces résultats, qui suggère une fonction délétère du récepteur de type périphérique aux benzodiazépines dans les processus de cancérisation, constitue une base pertinente pour la recherche de ligands synthétiques spécifiques du récepteur de type périphérique aux benzodiazépines capables d'en bloquer les effets.

Les composés peuvent donc être utilisés pour le traitement des tumeurs et cancers.

Les récepteurs de type périphérique aux benzodiazépines sont également présents au niveau de la peau et, à ce titre, les composés utilisables selon l'invention peuvent être utilisés pour la prophylaxie ou le traitement des stress cutanés.
Par stress cutané, on entend les différentes situations qui pourraient provoquer des dommages en particulier au niveau de l'épiderme, quel que soit l'agent qui provoque ce stress. Cet agent peut être interne et/ou externe à l'organisme, comme un agent chimique ou radicalaire, ou bien externe, comme un rayonnement ultraviolet.
Ainsi les composés utilisables selon l'invention sont destinés à prévenir et à lutter contre les irritations cutanées, les dartres, les érythèmes, les sensations dysesthésiques, les sensations d'échauffement, les prurits de la peau et/ou des muqueuses, le vieillissement et peuvent aussi être utilisés dans les désordres cutanés tels que, par exemple, le psoriasis, les maladies prurigineuses, l'herpès, les photodermatoses, les dermatites atopiques, les dermatites de contact, les lichens, les prurigos, les piqûres d'insectes, dans les fibroses et autres troubles de la maturation des collagènes, dans les désordres immunologiques ou encore dans des affections dermatologiques comme l'eczéma.

Les composés de l'invention peuvent également être utilisés pour la prévention et le traitement des maladies inflammatoires chroniques, notamment la polyarthrite rhumatoïde.

Selon un autre de ses aspects, l'invention a pour objet des compositions pharmaceutiques comprenant, en tant que principe actif, au moins un composé de formule générale (I). Ces compositions pharmaceutiques contiennent une dose efficace d'au moins un composé de l'invention, à l'état de base, de sel pharmaceutiquement acceptable, de solvat ou d'hydrate, et éventuellement associé à au mois un excipient pharmaceutiquement acceptable.
Lesdits excipients sont choisis selon la forme pharmaceutique et le mode d'administration souhaité, parmi les excipients habituels qui sont connus de l'Homme du métier.

Dans les compositions pharmaceutiques de l'invention pour l'administration orale, sublinguale, sous-cutanée, intramusculaire, intraveineuse, topique, locale, intratrachéale, intranasale, transdermique, rectale, intraocculaire, le principe actif de formule générale (I) ci-dessus, ou son sel, solvat ou hydrate éventuel, peut être administré sous forme unitaire d'administration, en mélange avec au moins un excipient pharmaceutique classique, aux animaux et aux êtres humains pour la prophylaxie ou le traitement des troubles et des maladies ci-dessus.

Les formes unitaires d'administration peuvent être, par exemple, des comprimés, des gélules, des granules, des poudres, des solutions ou suspensions orales ou injectables, des timbres transdermiques ("patch"), des formes d'administration sublinguale, buccale, intratrachéale, intraoculaire, intranasale, par inhalation, des formes d'administration topique, transdermique, sous-cutanée, intramusculaire ou intraveineuse, des formes d'administration rectale ou des implants. Pour l'administration topique on peut envisager des crèmes, gels, pommades, lotions ou collyres.
Ces formes galéniques sont préparées selon les méthodes usuelles des domaines considérés.

Lesdites formes unitaires sont dosées pour permettre une administration journalière de 0,001 à 20 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé répondant à la formule générale (I) 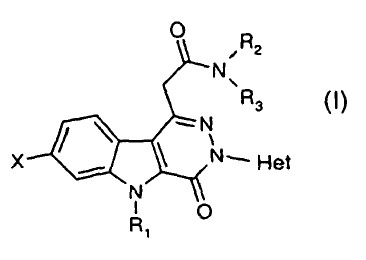 dans laquelle
X représente un atome d'hydrogène ou d'halogène,
R₁ représente un atome d'hydrogène ou un groupe (C₁-C₄)alkyle,
R₂ et R₃ représentent chacun, indépendamment l'un de l'autre, un atome d'hydrogène ou un groupe (C₁-C₄)alkyle, ou bien R₂ et R₃ forment, avec l'atome d'azote qui les porte, un groupe pyrrolidinyle, pipéridinyle, morpholinyle ou 4-(C₁-C₄)alkylpipérazinyle, et
Het représente un groupe hétéroaromatique de type pyridinyle, quinolinyle, isoquinolinyle, pyrimidinyle, pyrazinyle, pyridazinyle, le groupe hétéroaromatique pouvant porter un ou plusieurs atomes d'halogènes et/ou un ou plusieurs groupes (C₁-C₄)alkyle, (C₁-C₄)alcoxyle,
à l'état de base ou de sel d'addition à des acides, ainsi qu'à l'état d'hydrate ou de solvat.

2. Composé selon la revendication 1, **caractérisé en ce que** X représente un atome d'halogène.

3. Composé selon la revendication 1 ou 2, **caractérisé en ce que** R₁ représente un (C₁-C₄)alkyle.

4. Composé selon l'une quelconque des revendications 1 à 3, **caractérisé en ce que** R₂ et R₃ représentent chacun, indépendamment l'un de l'autre, un groupe (C₁-C₄)alkyle, ou bien R₂ et R₃ forment, avec l'atome d'azote qui les porte, un groupe pyrrolidinyle, ou 4-(C₁-C₄)alkylpipérazinyle.

5. Composé selon l'une quelconque des revendications 1 à 4, **caractérisé en ce que** Het représente un groupe hétéroaromatique de type pyridinyle pouvant porter un ou plusieurs atomes d'halogène et/ou un ou plusieurs groupes (C₁-C₄)alkyle, (C₁-C₄)alcoxyle.

6. Composé selon l'une quelconque des revendications 1 à 5, **caractérisé en ce que** X représente un atome de chlore et R₁ représente un groupe méthyle.

7. Procédé de préparation d'un composé de formule générale (I), 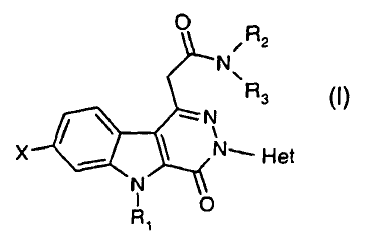 dans laquelle
X représente un atome d'hydrogène ou d'halogène,
R₁ représente un atome d'hydrogène ou un groupe (C₁-C₄)alkyle,
R₂ et R₃ représentent chacun, indépendamment l'un de l'autre, un atome d'hydrogène ou un groupe (C₁-C₄)alkyle, ou bien R₂ et R₃ forment, avec l'atome d'azote qui les porte, un groupe pyrrolidinyle, pipéridinyle, morpholinyle ou 4-(C₁-C₄)alkylpipérazinyle, et
Het représente un groupe hétéroaromatique de type pyridinyle, quinolinyle, isoquinolinyle, pyrimidinyle, pyrazinyle, pyridazinyle, le groupe hétéroaromatique pouvant porter un ou plusieurs atomes d'halogènes et/ou un ou plusieurs groupes (C₁-C₄)alkyle, (C₁-C₄)alcoxyle,
**caractérisé en ce que** l'on fait réagir le composé de formule générale (IV) 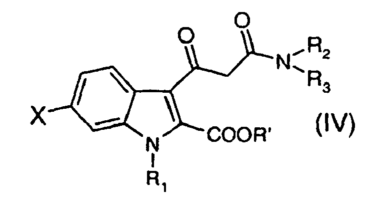 dans laquelle
X, R₁, R₂ et R₃ sont tels que définis ci-dessus,
R' représente un groupe (C₁-C₄)alkyle,
dans un solvant polaire en présence d'acide, avec une hétéroarylhydrazine.

8. Procédé selon la revendication 7, **caractérisé en ce que** le composé de formule générale (IV), 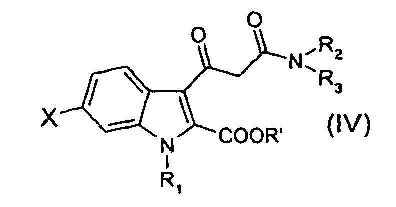 dans laquelle X, R₁, R₂, R₃ sont tels que définis dans la formule (I) selon la revendication 1 et R' représente un groupe (C₁-C₄)alkyle,
est préparé en faisant réagir un composé de formule générale (III), 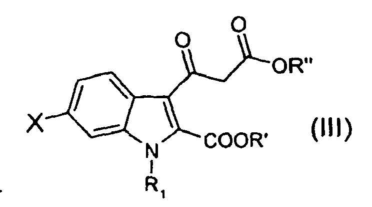 dans laquelle
X et R₁ sont tels que définis dans la formule (I) selon la revendication 1, R' représente un groupe (C₁-C₄)alkyle et R" représente un groupe (C₁-C₄)alkyle,
avec une amine de formule générale HNR₂R₃, dans laquelle R₂ et R₃ sont tels que définis dans la formule (I) selon la revendication 1, en présence d'un catalyseur comme la 4-(diméthylamino)pyridine.

9. Procédé de préparation d'un composé de formule générale (I), 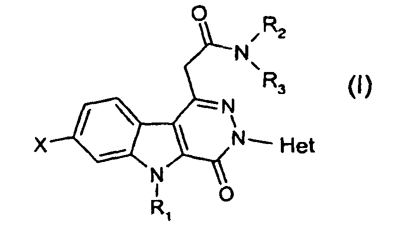 dans laquelle
X représente un atome d'hydrogène ou d'halogène,
R₁ représente un atome d'hydrogène ou un groupe (C₁-C₄)alkyle,
R₂ et R₃ représentent chacun, indépendamment l'un de l'autre, un atome d'hydrogène ou un groupe (C₁-C₄)alkyle, ou bien R₂ et R₃ forment, avec l'atome d'azote qui les porte, un groupe pyrrolidinyle, pipéridinyle, morpholinyle ou 4-(C₁-C₄)alkylpipérazinyle, et
Het représente un groupe hétéroaromatique de type pyridinyle, quinolinyle, isoquinolinyle, pyrimidinyle, pyrazinyle, pyridazinyle, le groupe hétéroaromatique pouvant porter un ou plusieurs atomes d'halogènes et/ou un ou plusieurs groupes (C₁-C₄)alkyle, (C₁-C₄)alcoxyle,
comprenant l'étape consistant à
réaliser une réaction de N-hétéroarylation sur un composé de formule générale (V) 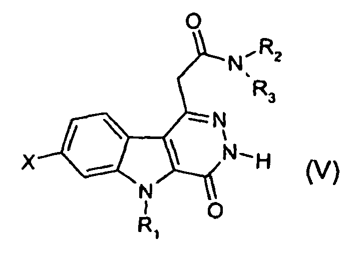 dans laquelle
X, R₁, R₂ et R₃ sont tels que définis ci-dessus,
en présence d'un halogénure d'hétéroaryle, ou bien d'un dérivé d'acide hétéroarylboronique et d'un sel métallique tel que un sel de cuivre.

10. Procédé selon la revendication 9, **caractérisé en ce que** composé de formule générale (V), 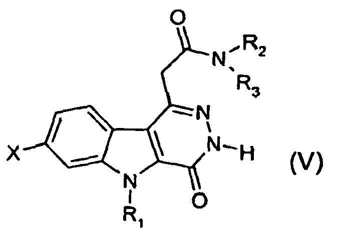 dans laquelle
X, R₁, R₂ et R₃ sont tels que définis dans la formule (I) selon la revendication 1,
est préparé en faisant réagir un composé de formule générale (IV), 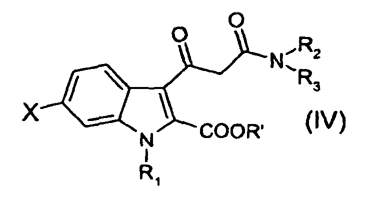 dans laquelle
X, R₁, R₂, R₃ sont tels que définis dans la formule (I) selon la revendication 1,
R' représente un groupe (C₁-C₄)alkyle,
avec de l'hydrazine en chauffant dans un solvant comme le toluène en présence d'une quantité catalytique d'acide.

11. Composé répondant à la formule générale (III) 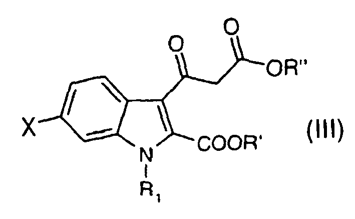 dans laquelle
X représente un atome d'hydrogène ou d'halogène,
R₁ représente un atome d'hydrogène ou un groupe (C₁-C₄)alkyle,
R' et R" représentent chacun, indépendamment l'un de l'autre, un groupe (C₁-C₄)alkyle.

12. Composé répondant à la formule générale (IV) 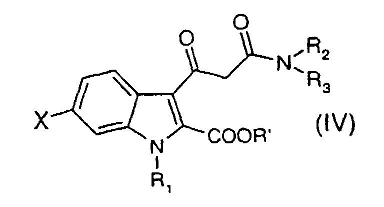 dans laquelle
X représente un atome d'hydrogène ou d'halogène,
R₁ représente un atome d'hydrogène ou un groupe (C₁-C₄)alkyle,
R' représente un groupe (C₁-C₄)alkyle,
R₂ et R₃ représentent chacun, indépendamment l'un de l'autre, un atome d'hydrogène ou un groupe (C₁-C₄)alkyle, ou bien R₂ et R₃ forment, avec l'atome d'azote qui les porte, un groupe pyrrolidinyle, pipéridinyle, morpholinyle ou 4-(C₁-C₄)alkylpipérazinyle.

13. Composé répondant à la formule générale (V) 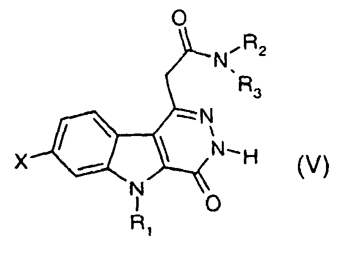 dans laquelle
X représente un atome d'hydrogène ou d'halogène,
R₁ représente un atome d'hydrogène ou un groupe (C₁-C₄)alkyle,
R₂ et R₃ représentent chacun, indépendamment l'un de l'autre, un atome d'hydrogène ou un groupe (C₁-C₄)alkyle, ou bien R₂ et R₃ forment, avec l'atome d'azote qui les porte, un groupe pyrrolidinyle, pipéridinyle, morpholinyle ou 4-(C₁-C₄)alkylpipérazinyle.

14. Composé selon l'une quelconque des revendications 1 à 6, ou sel pharmaceutiquement acceptable de ce dernier, ou hydrate ou solvat dudit composé, pour son utilisation comme médicament.

15. Composition pharmaceutique, **caractérisée en ce qu'**elle comprend au moins un composé de formule (I) selon l'une quelconque des revendications 1 à 6, ou un sel pharmaceutiquement acceptable, un hydrate ou un solvat de ce composé, éventuellement associé à au moins un excipient pharmaceutiquement acceptable.

## Claims

1. Compound of the general formula (I) 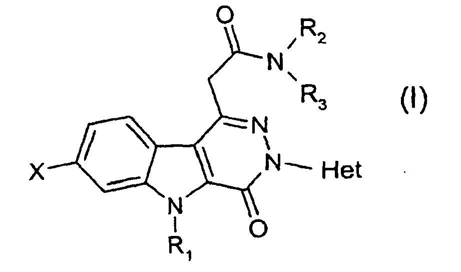 in which
X represents a hydrogen or halogen atom,
R₁ represents a hydrogen atom or a (C₁-C₄)alkyl group,
R₂ and R₃ each independently of one another represent a hydrogen atom or a (C₁-C₄)alkyl group, or else R₂ and R₃, together with the nitrogen atom bearing them, form a pyrrolidinyl, piperidinyl, morpholinyl or 4-(C₁-C₄)-alkylpiperazinyl group, and
Het represents a heteroaromatic group of pyridinyl, quinolinyl, isoquinolinyl, pyrimidinyl, pyrazinyl or pyridazinyl type which may carry one or more halogen atoms and/or one or more (C₁-C₄) alkyl and/or (C₁₋C₄)alkoxy groups,
in the form of the base or an addition salt with acids, or in the hydrate or solvate form.

2. Compound according to Claim 1, **characterized in that** X represents a halogen atom.

3. Compound according to Claim 1 or 2, **characterized in that** R₁ represents a (C₁-C₄)alkyl.

4. Compound according to any one of Claims 1 to 3, **characterized in that** R₂ and R₃, each independently of one another, represent a (C₁-C₄)alkyl group or else R₂ and R₃, together with the nitrogen atom bearing them, form a pyrrolidinyl or 4-(C₁-C₄)alkylpiperazinyl group.

5. Compound according to any one of Claims 1 to 4, **characterized in that** Het represents a heteroaromatic group of pyridinyl type which may carry one or more halogen atoms and/or one or more (C₁₋C₄)alkyl and/or (C₁-C₄)alkoxy groups.

6. Compound according to any one of Claims 1 to 5, **characterized in that** X represents a chlorine atom and R₁ represents a methyl group.

7. Process for preparing a compound of general formula (I), 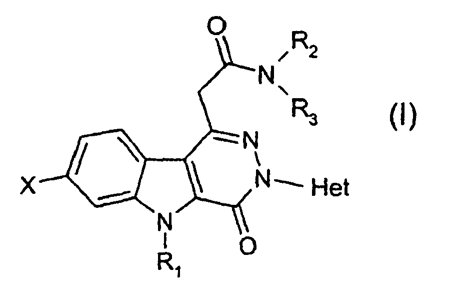 in which
X represents a hydrogen or halogen atom,
R₁ represents a hydrogen atom or a (C₁-C₄)alkyl group,
R₂ and R₃ each independently of one another represent a hydrogen atom or a (C₁-C₄)alkyl group, or else R₂ and R₃, together with the nitrogen atom bearing them, form a pyrrolidinyl, piperidinyl, morpholinyl or 4-(C₁-C₄)-alkylpiperazinyl group, and
Het represents a heteroaromatic group of pyridinyl, quinolinyl, isoquinolinyl, pyrimidinyl, pyrazinyl or pyridazinyl type which may carry one or more halogen atoms and/or one or more (C₁-C₄) alkyl and/or (C₁-C₄)-alkoxy groups,
**characterized in that** the compound of general formula (IV), 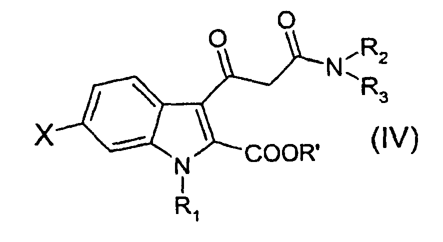 in which
X, R₁, R₂ and R₃ are as defined above,
R' represents a (C₁-C₄)alkyl group,
is reacted, in a polar solvent in the presence of acid, with a heteroarylhydrazine.

8. Process according to Claim 7, **characterized in that** the compound of general formula (IV), 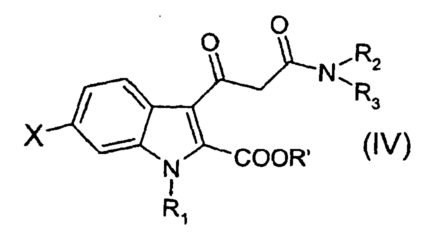 in which
X, R₁, R₂ and R₃ are as defined in formula (I) according to Claim 1, and R' represents a (C₁-C₄)alkyl group,
is prepared by reacting a compound of general formula (III), 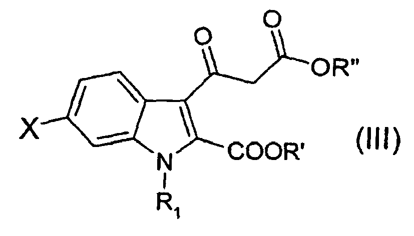 in which
X and R₁ are as defined in formula (I) according to Claim 1, R' represents a (C₁-C₄) alkyl group
and R" represents a (C₁-C₄) alkyl group,
with an amine of general formula HNR₂R₃, in which R₂ and R₃ are as defined in formula (I) according to Claim 1, in the presence of a catalyst such as 4-(dimethylamino)pyridine.

9. Process for preparing a compound of general formula (I), 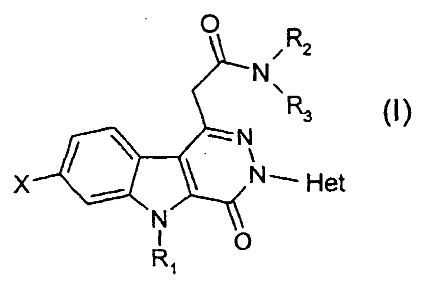 in which
X represents a hydrogen or halogen atom,
R₁ represents a hydrogen atom or a (C₁-C₄)alkyl group,
R₂ and R₃ each independently of one another represent a hydrogen atom or a (C₁-C₄) alkyl group, or else R₂ and R₃, together with the nitrogen atom bearing them, form a pyrrolidinyl, piperidinyl, morpholinyl or 4-(C₁-C₄)-alkylpiperazinyl group, and
Het represents a heteroaromatic group of pyridinyl, quinolinyl, isoquinolinyl, pyrimidinyl, pyrazinyl or pyridazinyl type which may carry one or more halogen atoms and/or one or more (C₁-C₄)alkyl and/or (C₁-C₄)-alkoxy groups,
comprising the step consisting in carrying out an N-heteroarylation reaction on a compound of general formula (V), 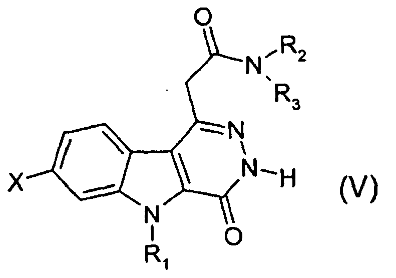 in which
X, R₁, R₂ and R₃ are as defined above,
in the presence of a heteroaryl halide, or else of a heteroarylboronic acid derivative and of a metal salt such as a copper salt.

10. Process according to Claim 9, **characterized in that** the compound of general formula (V), 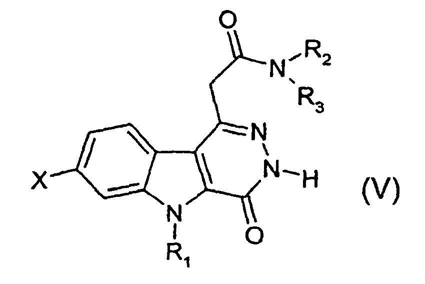 in which
X, R₁, R₂ and R₃ are as defined in formula (I) according to Claim 1,
is prepared by reacting a compound of general formula (IV), 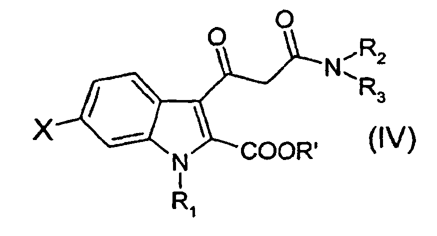 in which
X, R₁, R₂, R₃ are as defined in formula (I) according to Claim 1,
R' represents a (C₁-C₄) alkyl group,
with hydrazine by heating in a solvent such as toluene in the presence of a catalytic amount of acid.

11. Compound of the general formula (III) 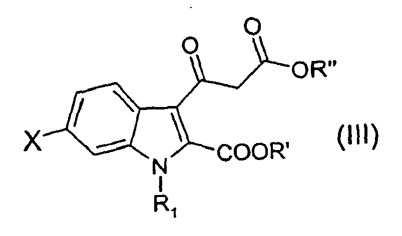 in which
X represents a hydrogen or halogen atom,
R₁ represents a hydrogen atom or a (C₁-C₄)alkyl group,
R' and R", each independently of one another, represent a (C₁-C₄) alkyl group.

12. Compound of the general formula (IV) 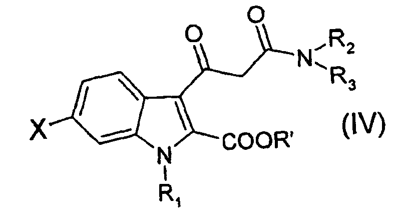 in which
X represents a hydrogen or halogen atom,
R₁ represents a hydrogen atom or a (C₁-C₄)alkyl group,
R' represents a (C₁-C₄) alkyl group,
R₂ and R₃, each independently of one another, represent a hydrogen atom or a (C₁-C₄)alkyl group, or else R₂ and R₃, together with the nitrogen atom bearing them, form a pyrrolidinyl, piperidinyl, morpholinyl or 4-(C₁₋C₄)alkylpiperazinyl group.

13. Compound of the general formula (V) 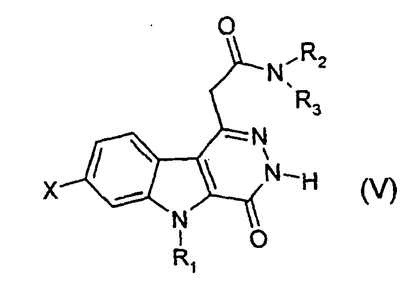 in which
X represents a hydrogen or halogen atom,
R₁ represents a hydrogen atom or a (C₁-C₄)alkyl group,
R₂ and R₃, each independently of one another, represent a hydrogen atom or a (C₁-C₄)alkyl group, or else R₂ and R₃, together with the nitrogen atom bearing them, form a pyrrolidinyl, piperidinyl, morpholinyl or 4-(C₁₋C₄)alkylpiperazinyl group.

14. Compound according to any one of Claims 1 to 6, or pharmaceutically acceptable salt thereof, or hydrate or solvate of said compound, for its use as medicinal product.

15. Pharmaceutical composition **characterized in that** it comprises at least one compound of formula (I) according to any one of Claims 1 to 6, or a pharmaceutically acceptable salt, a hydrate or a solvate of this compound, optionally combined with at least one pharmaceutically acceptable excipient.

## Patentansprüche

1. Verbindung, die der allgemeinen Formel (I) 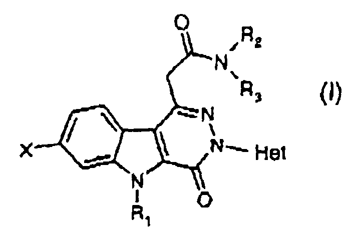 entspricht, worin
X ein Wasserstoff- oder Halogenatom darstellt,
R₁ ein Wasserstoffatom oder eine (C₁-C₄)-Alkylgruppe darstellt,
R₂ und R₃ jeweils unabhängig voneinander ein Wasserstoffatom oder eine (C₁-C₄)-Alkylgruppe darstellen oder aber R₂ und R₃ mit dem Stickstoffatom, das sie trägt, eine Pyrrolidinyl-, Piperidinyl-, Morpholinyl- oder 4-(C₁-C₄)-Alkylpiperazinylgruppe bilden und
Het eine heteroaromatische Gruppe vom Typ Pyridinyl, Chinolinyl, Isochinolinyl, Pyrimidinyl, Pyrazinyl, Pyridazinyl darstellt, wobei die heteroaromatische Gruppe ein oder mehrere Halogenatome und/oder eine oder mehrere (C₁-C₄)-Alkylgruppen, (C₁₋C₄)-Alkoxylgruppen tragen kann,
in Form einer Base oder eines Additionssalzes mit Säuren sowie in Form eines Hydrats oder eines Solvats.

2. Verbindung gemäß Anspruch 1, **dadurch gekennzeichnet, dass** X ein Halogenatom darstellt.

3. Verbindung gemäß Anspruch 1 oder 2, **dadurch gekennzeichnet, dass** R₁ ein (C₁-C₄)-Alkyl darstellt.

4. Verbindung gemäß einem der Ansprüche 1 bis 3, **dadurch gekennzeichnet, dass** R₂ und R₃ jeweils unabhängig voneinander eine (C₁-C₄)-Alkylgruppe darstellen oder aber R₂ und R₃ mit dem Stickstoffatom, das sie trägt, eine Pyrrolidinyl- oder 4-(C₁-C₄)-Alkylpiperazinylgruppe bilden,

5. Verbindung gemäß einem der Ansprüche 1 bis 4, **dadurch gekennzeichnet, dass** Het eine heteroaromatische Gruppe vom Typ Pyridinyl darstellt, die ein oder mehrere Halogenatome und/oder eine oder mehrere (C₁-C₄)-Alkylgruppen, (C₁-C₄)-Alkoxylgruppen tragen kann.

6. Verbindung gemäß einem der Ansprüche 1 bis 5, **dadurch gekennzeichnet, dass** X ein Chloratom darstellt und R₁ eine Methylgruppe darstellt.

7. Verfahren zur Herstellung einer Verbindung der allgemeinen Formel (I) 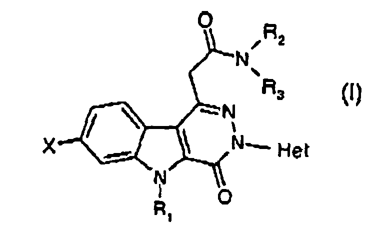 worin
X ein Wasserstoff- oder Halogenatom darstellt,
R₁ ein Wasserstoffatom oder eine (C₁-C₄)-Alkylgruppe darstellt,
R₂ und R₃ jeweils unabhängig voneinander ein Wasserstoffatom oder eine (C₁-C₄)-Alkylgruppe darstellen oder aber R₂ und R₃ mit dem Stickstoffatom, das sie trägt, eine Pyrrolidinyl-, Piperidinyl-, Morpholinyl- oder 4-(C₁-C₄)-Alkylpiperazinylgruppe bilden und
Het eine heteroaromatische Gruppe vom Typ Pyridinyl, Chinolinyl, Isochinolinyl, Pyrimidinyl, Pyrazinyl, Pyridazinyl darstellt, wobei die heteroaromatische Gruppe ein oder mehrere Halogenatome und/oder eine oder mehrere (C₁-C₄)-Alkylgruppen, (C₁₋C₄)-Alkoxylgruppen tragen kann,
**dadurch gekennzeichnet, dass** man die Verbindung der allgemeinen Formel (IV) 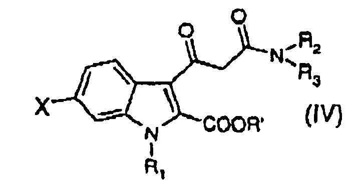 worin
X, R₁, R₂ und R₃ wie oben definiert sind,
R' eine (C₁-C₄)-Alkylgruppe darstellt,
in einem polaren Lösungsmittel in Gegenwart von Säure mit einem Heteroarylhydrazin umsetzt.

8. Verfahren gemäß Anspruch 7, **dadurch gekennzeichnet, dass** die Verbindung der allgemeinen Formel (IV) 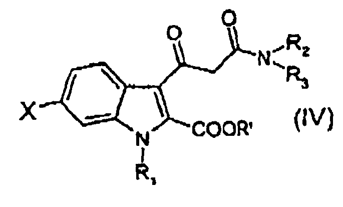 worin
X, R₁, R₂, R₃ wie in der Formel (I) gemäß Anspruch 1 definiert sind und R' eine (C₁₋C₄)-Alkylgruppe darstellt,
hergestellt wird, indem man eine Verbindung der allgemeinen Formel (III) 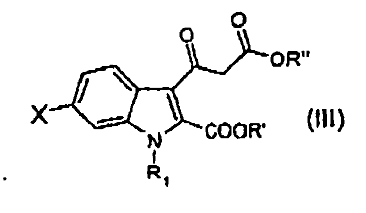 worin
X und R₁ wie in der Formel (I) gemäß Anspruch 1 definiert sind, R' eine (C₁-C₄)-Alkylgruppe darstellt und R" eine (C₁-C₄)-Alkylgruppe darstellt,
mit einem Amin der allgemeinen Formel HNR₂R₃, worin R₂ und R₃ wie in der Formel (I) gemäß Anspruch 1 definiert sind, in Gegenwart eines Katalysators, wie 4-(Dimethylamino)pyridin, umsetzt.

9. Verfahren zur Herstellung einer Verbindung der allgemeinen Formel (I) 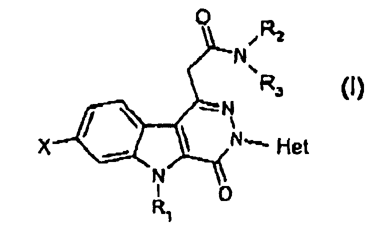 worin
X ein Wasserstoff- oder Halogenatom darstellt,
R₁ ein Wasserstoffatom oder eine (C₁-C₄)-Alkylgruppe darstellt,
R₂ und R₃ jeweils unabhängig voneinander ein Wasserstoffatom oder eine (C₁-C₄)-Alkylgruppe darstellen oder aber R₂ und R₃ mit dem Stickstoffatom, das sie trägt, eine Pyrrolidinyl-, Piperidinyl-, Morpholinyl- oder 4-(C₁-C₄)-Alkylpiperazinylgruppe bilden und
Het eine heteroaromatische Gruppe vom Typ Pyridinyl, Chinolinyl, Isochinolinyl, Pyrimidinyl, Pyrazinyl, Pyridazinyl darstellt, wobei die heteroaromatische Gruppe ein oder mehrere Halogenatome und/oder eine oder mehrere (C₁-C₄)-Alkylgruppen, (C₁₋C₄)-Alkoxylgruppen tragen kann,
das den Schritt umfasst, der daraus besteht,
eine N-Heteroarylierungsreaktion an einer Verbindung der allgemeinen Formel (V) 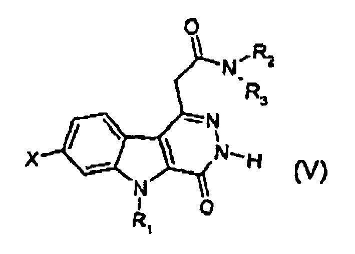 worin
X, R₁, R₂ und R₃ wie oben definiert sind,
in Gegenwart eines Heteroarylhalogenids oder aber eines Heteroarylboronsäurederivats und eines Metallsalzes, wie ein Kupfersalz, durchzuführen.

10. Verfahren gemäß Anspruch 9, **dadurch gekennzeichnet, dass** die Verbindung der allgemeinen Formel (V) 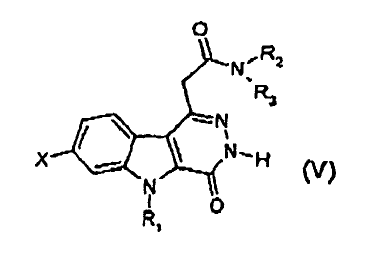 worin
X, R₁, R₂ und R₃ wie in der Formel (I) gemäß Anspruch 1 definiert sind,
hergestellt wird, indem man eine Verbindung der allgemeinen Formel (IV) 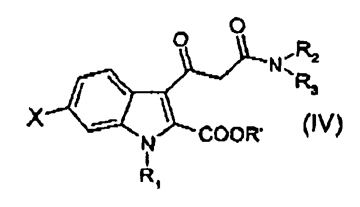 worin
X, R₁, R₂, R₃ wie in der Formel (I) gemäß Anspruch 1 definiert sind,
R' eine (C₁-C₄)-Alkylgruppe darstellt,
mit Hydrazin umsetzt, indem man in einem Lösungsmittel, wie Toluen, in Gegenwart einer katalytischen Menge an Säure erhitzt.

11. Verbindung, die der allgemeinen Formel (III) 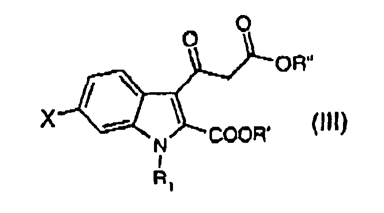 entspricht, worin
X ein Wasserstoff- oder Halogenatom darstellt,
R₁ ein Wasserstoffatom oder eine (C₁-C₄)-Alkylgruppe darstellt,
R' und R" jeweils unabhängig voneinander eine (C₁-C₄)-Alkylgruppe darstellen.

12. Verbindung, die der allgemeinen Formel (IV) 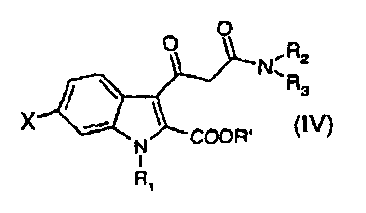 entspricht, worin
X ein Wasserstoff- oder Halogenatom darstellt,
R₁ ein Wasserstoffatom oder eine (C₁-C₄)-Alkylgruppe darstellt,
R' eine (c₁-C₄)-Alkylgruppe darstellt,
R₂ und R₃ jeweils unabhängig voneinander ein Wasserstoffatom oder eine (C₁-C₄)-Alkylgruppe darstellen oder aber R₂ und R₃ mit dem Stickstoffatom, das sie trägt, eine Pyrrolidinyl-, Piperidinyl-, Morpholinyl- oder 4-(C₁-C₄)-Alkylpiperazinylgruppe bilden.

13. Verbindung, die der allgemeinen Formel (V) 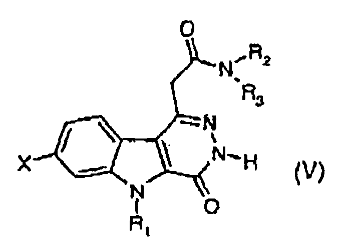 entspricht, worin
X ein Wasserstoff- oder Halogenatom darstellt,
R₁ ein Wasserstoffatom oder eine (C₁-C₄)-Alkylgruppe darstellt,
R₂ und R₃ jeweils unabhängig voneinander ein Wasserstoffatom oder eine (C₁-C₄)-Alkylgruppe darstellen oder aber R₂ und R₃ mit dem Stickstoffatom, das sie trägt, eine Pyrrolidinyl-, Piperidinyl-, Morpholinyl- oder 4-(C₁-C₄)-Alkylpiperazinylgruppe bilden.

14. Verbindung gemäß einem der Ansprüche 1 bis 6 oder pharmazeutisch unbedenkliches Salz dieser Letzteren oder Hydrat oder Solvat besagter Verbindung für ihre Verwendung als Arzneimittel.

15. Pharmazeutische Zusammensetzung, **dadurch gekennzeichnet, dass** sie wenigstens eine Verbindung der Formel (I) gemäß einem der Ansprüche 1 bis 6 oder ein pharmazeutisch unbedenkliches Salz, eln Hydrat oder ein Solvat dieser Verbindung, gegebenenfalls kombiniert mit wenigstens einem pharmazeutisch unbedenklichen Hilfsstoff, umfasst.
